# EUROPEAN PATENT APPLICATION

(11) **EP 4 480 403 A1**
(43) Date of publication of application: **25.12.2024**
(21) Application number: 23181116.7
(22) Date of filing: 23.06.2023
(51) Int. Cl.: A61B 5/026

(54) **PERSONAL CARE DEVICE WHICH MEASURES CAPILLARY REFILL TIME**

(71) Applicant: Koninklijke Philips N.V., 5656 AG Eindhoven (NL)
(72) Inventor: GOTTENBOS, Bart, Eindhoven (NL); GERHARDT, Lutz Christian, Eindhoven (NL); MENA BENITO, Maria Estrella, 5656AG Eindhoven (NL); KOOIJMAN, Gerben, Eindhoven (NL); JOHNSON, Mark Thomas, Eindhoven (NL)
(74) Representative: Philips Intellectual Property & Standards

(57) **Abstract**

A personal care device applies a force to body tissue. The force applied is detected in use of the personal care device. If sufficient force has been applied to the body tissue to enable a measurement of capillary refill time, CRT, then a capillary refill time is derived from spectral, i.e., color, changes detected by an optical sensor. In this way, the processing to measure CRT is only carried out when it is likely to enable a successful CRT measurement.

## Description

### FIELD OF THE INVENTION

This invention relates to personal care devices, and in particular personal care devices which enable measurement of capillary refill time.

### BACKGROUND OF THE INVENTION

When a force is applied to the body tissue, such as the skin or gums, the blood is pressed out, changing the tissue color (making the pressed spot less red). When the pressure is released, the blood quickly comes back, refilling the blood capillaries in the tissue. The time this takes is the capillary refill time, CRT.

CRT is often clinically measured on the fingers to enable a quick assessment of the condition of an ill patient, ranging from dehydration to life threatening sepsis. Also, in a home setting, CRT monitoring would be valuable, especially in at-risk groups (e.g., infants, elderly, chronically ill), as it could indicate dehydration or poor breathing patterns. Breathing irregularity may for example signal the onset of an asthma attack.

Currently CRT is often measured in a subjective way, depending on the skills, experience, and perception of the person assessing CRT. By using an automated approach, e.g., through the use of an optical sensor or camera, this subjective aspect can be removed from the CRT measurement, and it would become objective and reproducible. For a practical implementation for home use, it would be beneficial if CRT could be unobtrusively and automatically measured while performing another routine.

It has been proposed in EP 3398503 to measure CRT using an oral care device on the gums. The proposed solution requires a dedicated means of applying pressure to the gums (such as an air jet, water jet, mechanical peg or pin).

There is therefore a need for a simpler solution to the measurement of CRT.

Personal care devices, such as power toothbrushes, increasingly have onboard sensors such as cameras for various functions, as such camera sensors are becoming very low cost. It would be desirable to enable use of existing sensors to perform a CRT measurement.

### SUMMARY OF THE INVENTION

The invention is defined by the claims.

According to examples in accordance with an aspect of the invention, there is provided a personal care device for implementing a personal care function which involves application of force to body tissue, the device comprising:
a force sensing means for detecting a force applied by the personal care device to the body tissue in use of the personal care device to perform the personal care function;
an optical sensor; and
a processing system configured to:
   determine if sufficient force has been applied to the body tissue to enable a measurement of capillary refill time from the force detected by the force sensor; and
   after said sufficient force has been applied, determine a capillary refill time from spectral changes in reflected light from the tissue detected by the optical sensor.

This personal care device makes use of an optical sensor and a force sensing means to enable CRT to be determined. These sensors are typically present in many different personal care devices such as powered toothbrushes, so in many cases the invention can be implemented using typical hardware already present in a device. The force applied to the body tissue is applied by the user of the device when they perform the personal care function, e.g., brushing the teeth and gums.

The force sensing means may directly measure a force, or it may measure a property which is correlated with the force applied. It may for example be an optical sensor. CRT is measured using a camera or similar optical sensor while the personal care device is passed over the body tissue (e.g., gums or skin). For good reliability of the CRT measurement, the processing system checks whether sufficient force is applied by the user to fully remove the blood from the tissue.

The optical sensor for example comprises a camera. In this way, a measurement location can be present in multiple sequential images even when the personal care device is moved between the images. Thus, the field of view of each image includes a measurement location so that spectral changes, i.e., color changes, over time can be tracked at that measurement location.

The optical sensor for example has a field of view adjacent a location where force is applied by the personal care device to the body tissue. This is suitable when the head to which force is applied is moved over the body tissue, as is the case for tooth brushing. Thus, the field of view includes a location where capillary refill takes place after a force has been applied.

The optical sensor may (instead or as well) have a field of view including a location where force is applied by the personal care device to the body tissue. This is suitable when a force is applied and then removed from a same location. This is for example the case for a breast pump. In such a case, the optical sensor may need to obtain an image through the component that applies the force to the body tissue (e.g., a breast shield in the case of a breast pump).

Using optical sensing of the location at which force is applied, the processing system may be further configured to determine a capillary emptying time. The same approach is taken of measuring a change in reflection spectrum, i.e., color, over time.

The personal care device for example further comprises a motion sensor for detecting motion and/or motion direction of the personal care device, wherein the processing system is further configured to determine if the motion and/or motion direction of the personal care device, after said sufficient force has been applied, is suitable for measurement of capillary refill time.

This is particularly suitable for a device which is moved over the body tissue. In this way, the motion sensor (e.g., inertial measurement unit) checks whether the correct motion is applied to the device to record the CRT after passing of the device. An additional check can be added to make sure the device is positioned on the proper tissue location (e.g., on the gums in oral applications).

In one approach, the processing system is configured to determine a capillary refill time by identifying a same measurement location in a series of images captured by the optical sensor, and monitoring spectral changes of the measurement location over time from the series of images.

The processing system may in this case be configured to identify the same measurement location based on motion sensing and/or feature recognition.

In another approach, the processing system is configured to determine a capillary refill time by monitoring spectral changes between different body tissue areas, corresponding to a path along which force is applied by the personal care device, within a single image captured by the optical sensor.

In this way, a single image provides a snapshot of spatial capillary refill conditions. Knowledge of the path followed by the personal care device enables the time at which pressure was applied to different body tissue locations to be determined, so that CRT can be determined. This reduces the amount of data processing needed.

The personal care device may comprise a triggering system for triggering the optical sensor based on motion of the personal care device, wherein the processing arrangement is configured to process images of a measurement location taken before and after a force has been applied by the personal care device.

The optical measurement before and after force is applied, for example in front of and behind a moving toothbrush head, can be used to obtain a baseline value for the CRT measurement.

The personal care device may comprise a toothbrush having a brush head, wherein the optical sensor comprises a camera with a field of view of the gums behind and/or in front of the brush head.

In this way, the field of view is located where the tissue which has just been brushed will be present (depending on whether the brush has been moved forward or backward). The processing system may then be further configured to:
determine if the brush head has moved in a direction away from the optical sensor; and
determine the capillary refill time when the brush head has moved in said direction

The direction away from the optical sensor is for example along the direction of the handle. Movement in a direction away from the optical sensor means the tissue is in the field of view of the optical sensor from which a force has just been released. By using the motion sensing in this way, the amount of data processing required is reduced as only under the correct circumstances a CRT measurement is performed.

The invention also provides a method for determining a capillary refill time, comprising:
receiving a detected force applied by a personal care device to body tissue in use of the personal care device to perform a personal care function which involves application of force to the body tissue;
receiving an optical sensor output of the body tissue;
determining if sufficient force has been applied to the body tissue to enable a measurement of capillary refill time from the detected force; and
after said sufficient force has been applied, determining a capillary refill time from spectral changes of the optical sensor output.

The invention also provides a computer program comprising computer program code which is adapted, when said program is run on a computer, to implement the method defined above.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 shows a toothbrush which measures CRT;
Fig. 2 shows a method of measuring CRT using a toothbrush; and
Fig. 3 shows an optical sensor arrangement with two sensors.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a personal care device which applies a force to body tissue. The force applied is detected in use of the personal care device. If sufficient force has been applied to the body tissue to enable a measurement of capillary refill time, CRT, then a capillary refill time is derived from spectral, i.e., color, changes detected by an optical sensor. In this way, the processing to measure CRT is only carried out when it is likely to enable a successful CRT measurement.

The invention may be applied to any personal care device which applies a force to body tissue (in particular, body tissue with blood vessels), wherein that force is a result of the normal operation of the device. The device may be static so it applies a periodic force to a fixed location (e.g., a breast pump) or it may be a device which is moved over the body tissue (e.g., a toothbrush, massage device etc.).

The invention will be explained in detail with reference to the particular example of a powered toothbrush.

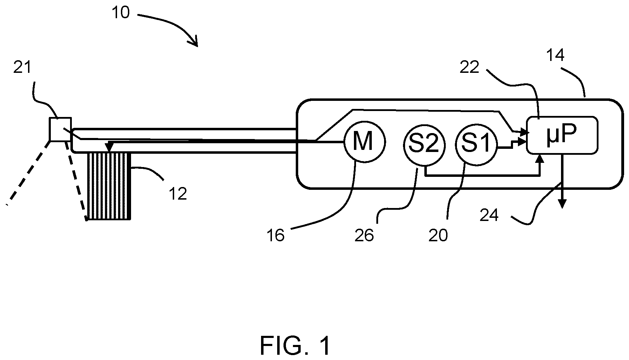

Fig. 1 shows a powered toothbrush 10 as an example of a personal care device. Thus, brushing the teeth is an example of a personal care function which involves application of force to body tissue, the body tissue of interest (having blood vessels) being the gums.

The toothbrush has a driven head 12 and a handle 14, with an actuator 16 in the handle for driving the head. The actuator in one example is a motor and the head is driven to rotate. A force sensor 20 detects a force applied by the toothbrush to the gums in use of the toothbrush. The force sensor measures the force the user applies in the normal direction towards the teeth and gums.

An optical sensor 21 is able to monitor the spectrum of reflected light from the gums, i.e., a color of the gums. A processing system, schematically shown as processor 22, determines if sufficient force has been applied to the body tissue to enable a measurement of capillary refill time from the force detected by the force sensor. Only when a sufficient force has been applied, a capillary refill time is determined from color changes detected by the optical sensor. The capillary refill time is output as shown by 24.

Fig. 1 also shows a motion sensor 26. The motion sensor is for example an inertial measurement unit, IMU, to identify position and motion. When motion sensing is used, not only does the force need to be above a required threshold, but also the brush head needs to move in a desired direction (e.g., in the direction of the handle) in order for a CRT measurement to be triggered.

The CRT is obtained by measuring the change of gum color over time, for example by using image sensor RGB channels. For example, a ratio of red to green or red to blue intensity levels will give a good contrast to monitor blood inflow.

Only an optical sensor needs to be added to enable the CRT measurement, and there is no need for a dedicated force-generating module. The user generates the force and the existing brush sensors are used to check that the force, and in preferred examples also the motion, are suitable.

The sensing makes the CRT measurement reliable, but also reduces the amount of data processing required as only under the correct circumstances a CRT measurement will be performed.

For the toothbrush example, the optical sensor (e.g., low-cost camera) has a field of view which corresponds to the gum location which has been passed by the bristle field of the toothbrush head. The color change rate of the gums from a lighter shade of pink to a darker shade of pink is monitored to enable the CRT to be derived.

The positioning and field of view of the optical sensor is such that the CRT is measurable. The CRT is typically around 1 second so that, at a typical toothbrush translation speed of 5mm/s, a field of view of 10mm length is able to monitor for 2s after the toothbrush has passed.

The reliability of the CRT measurement is improved by using sensor inputs from brush sensors, e.g., the brushing force, location, orientation, acceleration or from actuator signals inherent in such a device.

It has been demonstrated by experiment that a normal brushing pressure is sufficient to cause bleaching of the gums so that after passing of the bristles, a CRT can be determined.

The optical sensor may be implemented in various ways, but the positioning is such that the optical sensor captures a field of view including the tissue where, in the typical use case, the pressure is relieved. For example, the field of view is adjacent a location where force is applied by the toothbrush, such as beyond the distal bristles along the direction of the handle (as shown in Fig. 1). A typical user behavior for a power toothbrush is to move in a fluent motion from distal to frontal teeth.

Optionally, it is also possible to capture in the field of view the pressurized area, as this could provide an additional clinically relevant measurement; the capillary emptying time. For this purpose, the contact material with the body tissue should be transparent for the optical sensor, which could for example easily be embodied in a shaver or epilation device but is less evident in a toothbrush.

In brushes, shavers and epilators the users move the device, and this causes the release of the pressure. Other devices such as a breast pump or breast shield may not be moved, but by the personal care function itself the pressure is periodically released.

The optical sensor may be a RGB camera, but also other optical sensors capable of following the tissue redness in time at a specific spot of the tissue could be used. Since in many examples (including the example of a toothbrush) the sensor moves with the device, the measured spot also moves in the field of view. An algorithm runs on the processor 22 to determine the color of the spot of interest in time. For this purpose, a same measurement location may be identified based on motion sensing and/or feature recognition. The feature recognition for example identifies surrounding features, e.g., the shape of the gum line, or it may include information on the motion velocity from motion sensors and the size of the field of view (hence the distance of the optical sensor from the tissue).

As mentioned above, the conditions are determined to be appropriate for a reliable CRT measurement at least based on the force the user applies with the device on the tissue, as a minimum force will be required to bleach the tissue in the first place. Typical brushing forces are of the order of 1 to 2N. The minimum force to bleach the gum tissue will be lower than that, so that a suitable threshold force may be 1.0N or even 0.5N for a regular brush head size.

For smaller brush heads the threshold may be chosen to be lower. Thus, the threshold is for example in the range 0.2N to 1N.

A modification is that the device itself may determine the required threshold force for a particular user over a few uses, by finding the force at which the tissue becomes maximally bleached. This force could then even differ per location, as the local anatomy (e.g., gum thickness) may influence this bleaching force.

In user-translated devices such as toothbrushes and shavers, further motion data may be used to make sure that the device is moving between a certain minimum and maximum velocity, and that it is moving in the correct direction, in particular with the optical sensor at the tail of the movement.

A typical velocity range for a brush may be 1 to 10mm/s. Above the 10mm/s the field of view from the camera may not be sufficient to follow the spot for at least 2 seconds (e.g., 20mm long field of view). Below 1mm/s the bristles may not release the pressure fast enough to obtain a reliable starting point of the CRT measurement.

In the toothbrush use case, there may also be a situation that the brush is not on the gums, e.g., it could be on the occlusal surfaces. Position detection may be used as input as well to avoid recordings when there is no gum present at the measurement location. The optical sensor may also aid in position sensing by recognizing certain anatomical features or colors.

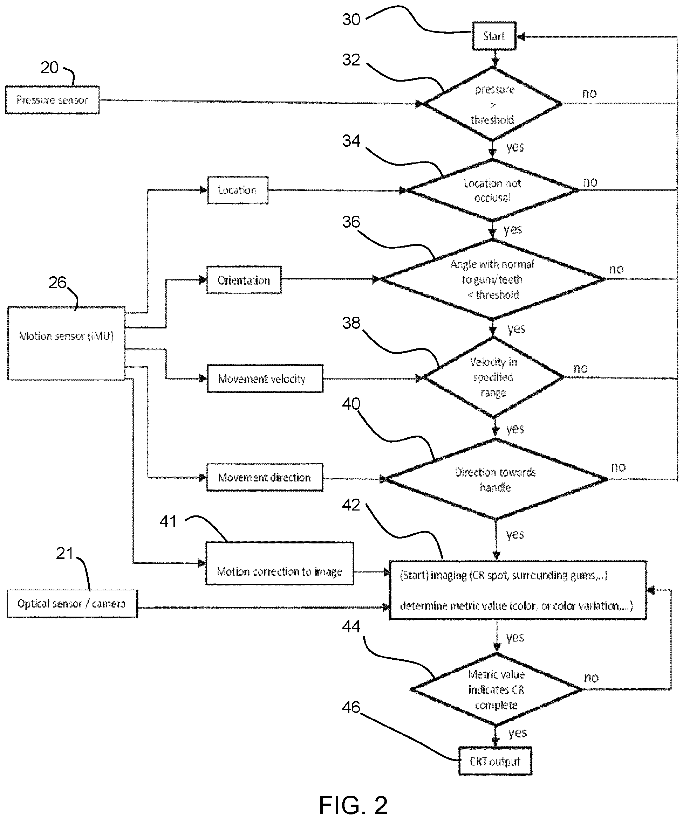

Fig. 2 shows a method for determining whether a reliable CRT measurement can be made in a toothbrush embodiment.

The method starts in step 30.

In step 32 it is determined if the applied force (or equivalently pressure) meets the threshold. If not, the method returns to the start. The force is measured by the force sensor 20.

In step 34 it is checked if the toothbrush location is not occlusal. If is occlusal, the method returns to the start.

In step 36 it is checked if the toothbrush orientation is suitable, hence close to the normal with respect to the gum surface. If the orientation is too far from the normal, the method returns to the start.

In step 38 it is checked if the toothbrush velocity is with a desired range. If the velocity is outside the range, the method returns to the start.

In step 40 the motion direction is determined, and checked if it is parallel with the handle axis and in the correct direction so that the optical sensor is at the tail of the applied force. If the motion is not correct, the method returns to the start.

These orientation and movement measurements are obtained from the motion sensor 26.

Motion correction is applied to the image in step 41 so that the color analysis is applied to the same measurement location in successive images.

In step 42, the imaging starts using the optical sensor 21. A metric color value is monitored. The color change determines when the capillary refill is complete in step 44 and when it is complete, the CRT is determined in step 46. The imaging of step 42 is continued until the capillary refill is complete.

In the examples above, the change in color over time of the gums is used for measurement of CRT. This requires a longitudinal series of images which has the disadvantage that a relatively large amount of data needs to be collected and analyzed.

An alternative approach is to assess the CRT using just a single snapshot of the gums. Specifically, by using a camera with a wide field of view and acquiring a wide view camera image in situations where the toothbrush moves at a low speed, a single snapshot can serve as a time-line to assess CRT. The position in the image away from the bristles in the axial direction along the brush is a representation of the time after applying (or indeed after releasing) the pressure of the bristles on the gum.

As such, a line scan in this direction of brush motion gives a characteristic profile for CRT with the color of the gum at t=0 sec being imaged in the snapshot closest to brush and t= (distance/velocity) seconds being imaged in the snapshot furthest from the brush.

In another approach to enable a reduction in data volume, a narrow image (a few lines of a full camera image) at the center of the full image may be sufficient to allow determination of the CRT.

Yet another approach for reducing data volume is to use motion triggered imaging. For example, imaging may be performed only during backward brushing for the case where the optical sensor is positioned facing the distal portion of the brush head (or alternatively during forward brushing when camera is positioned facing the proximal portion). Thus, the image capture only takes place when the optical sensor is within the motion tail of the toothbrush head.

The presence of toothpaste or toothpaste foam results in a highly scattering optical medium which will partially mask the gum colorations required to assess CRT. It is therefore beneficial to assess only images in which toothpaste is absent (at least in the regions of interest in the image). Toothpaste may be identified by image analysis so that images can be rejected with toothpaste in the wrong part (i.e., along the line scan described above).

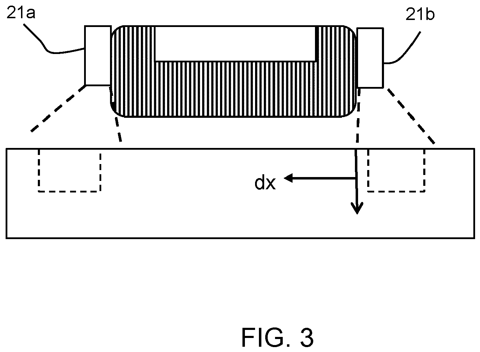

The example above has a single optical sensor. However, there may be optical sensors 21a, 21b at the front and back of the toothbrush head as shown in Fig. 3. This can be used to provide a reference i.e., baseline for calibrating the CRT measurement.

For a reliable and reproducible CRT measurement, a well-defined baseline or reference CRT value is required. The arrangement of Fig. 3 is able to image and measure the same measurement location before and after the brush has passed, using an imaging system configuration that is triggered by a motion signal, as well as a differential imaging algorithm. The motion direction of the brush is used to allow for the CRT reference measurement and re-image the same location when the brush has travelled a distance dx that is larger than the length of the brush head.

The imaging system (of the two optical sensors in this example) may be separable from the brush head and can be part of a click-on unit.

The method described below can be used to determine baseline value for CRT measurement. For explaining the method, the following values are defined:
L = width or length of brush head exerting a pressure/force on tissue
dx = distance travelled by toothbrush during brushing

The method comprises the following steps:
(i) Determine the motion direction via the motion sensor (e.g., IMU) and activate either imaging using optical sensor 21a or 21b accordingly.
(ii) Assuming motion is towards the left in Fig. 3, then start acquiring images with optical sensor 21a and determine a baseline tissue color (BTC0) in the L*a*b* color space or determine a baseline blood perfusion (BBP0).
(iii) Once the distance travelled by brush dx > L, then activate optical sensor 21b (which could be just a diode or waveguide connected to a central camera) to image the same area where baseline measurement was done.
(iv) Perform differential image analysis: Image from optical sensor 21a vs. the image sequence from optical sensor 21b.
(v) Determine from the image sequence from optical sensor 21a, the time t (= number of frames times frame rate) until the image color (for the same tracked measurement location) is equal to the baseline tissue color (BTC0) in the image from optical sensor 21a. This is the capillary refill time.

Motion direction triggered imaging may also be performed with only one optical sensor during back and forth stroke brushing, for providing a reference/baseline and to calibrate the CRT

In this case, an image baseline or reference gum color is obtained during a distal brushing direction and imaging the same gum location during the back-stroke motion (usually 0.5-1 Hz frequency) in the mesial direction is used for CRT calculation.

The invention is of interest for dental appliances generally, such as toothbrushes, oral irrigators and brushing mouthpieces, in that case targeting measurement of CRT on the gums. Professional dental appliances may also include such a sensing feature.

The invention may however be applied to other devices on the skin or lips such as shavers, epilators or infant feeding. It may also be applied to the skin at other areas of the body, for example during use of a breast pump.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

The mentioned force sensor can be a classical force sensing unit such as typically provided in power toothbrushes to guide users not to use too much force on the gums but is also referring to any means to determine the used force of the device on the tissue. For example, pressure measurement units may be used or an analysis of the toothbrush bristle bending using the embedded camera. Even the increased tissue whiteness measured by the optical sensor may be such a means to determine the applied force.

Functions implemented by a processor may be implemented by a single processor or by multiple separate processi.ng units which may together be considered to constitute a "processor". Such processing units may in some cases be remote from each other and communicate with each other in a wired or wireless manner.

The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems. (optional)

If the term "adapted to" is used in the claims or description, it is noted the term "adapted to" is intended to be equivalent to the term "configured to". If the term "arrangement" is used in the claims or description, it is noted the term "arrangement" is intended to be equivalent to the term "system", and vice versa.

Any reference signs in the claims should not be construed as limiting the scope.

## Claims

1. A personal care device (10) for implementing a personal care function which involves application of force to body tissue, the device comprising:
a force sensing means (20) for detecting a force applied by the personal care device to the body tissue in use of the personal care device to perform the personal care function;
an optical sensor (21); and
a processing system (22) configured to:
determine if sufficient force has been applied to the body tissue to enable a measurement of capillary refill time from the force detected by the force sensor; and
after said sufficient force has been applied, determine a capillary refill time (24) from spectral changes in reflected light from the tissue detected by the optical sensor.

2. The personal care device of claim 1, wherein the optical sensor (21) comprises a camera.

3. The personal care device of any one of claims 1 to 2, wherein the optical sensor (21) has a field of view adjacent a location where force is applied by the personal care device to the body tissue.

4. The personal care device of any one of claims 1 to 3, wherein the optical sensor (21) has a field of view including a location where force is applied by the personal care device to the body tissue.

5. The personal care device of claim 4, wherein the processing system (22) is further configured to determine a capillary emptying time.

6. The personal care device of any one of claims 1 to 5, further comprising a motion sensor (26) for detecting motion and/or motion direction of the personal care device, wherein the processing system (22) is further configured to determine if the motion and/or motion direction of the personal care device, after said sufficient force has been applied, is suitable for measurement of capillary refill time.

7. The personal care device of any one of claims 1 to 6, wherein the processing system (22) configured to determine a capillary refill time by identifying a same measurement location in a series of images captured by the optical sensor, and monitoring spectral changes of the measurement location over time from the series of images.

8. The personal care device of claim 7, wherein the processing system (22) is configured to identify the same measurement location based on motion sensing and/or feature recognition.

9. The personal care device of any one of claims 1 to 6, wherein the processing system (22) is configured to determine a capillary refill time by monitoring spectral changes between different body tissue areas, corresponding to a path along which force is applied by the personal care device, within a single image captured by the optical sensor.

10. The personal care device of any one of claims 1 to 9, comprising a triggering system for triggering the optical sensor based on motion of the personal care device, wherein the processing arrangement is configured to process images of a measurement location taken before and after a force has been applied by the personal care device.

11. The personal care device of claim 10, wherein the processing system (22) is configured to track a measurement location.

12. The personal care device of any one of claims 1 to 11, comprising a toothbrush having a brush head, wherein the optical sensor comprises a camera with a field of view of the gums behind and/or in front of the brush head.

13. The personal care device of claim 12, wherein the processing system is further configured to:
determine if the brush head has moved in a direction away from the optical sensor; and
determine the capillary refill time when the brush head has moved in said direction.

14. A method for determining a capillary refill time, comprising:
receiving a detected force applied by a personal care device to body tissue in use of the personal care device to perform a personal care function which involves application of force to the body tissue;
receiving an optical sensor output of the body tissue;
determining if sufficient force has been applied to the body tissue to enable a measurement of capillary refill time from the detected force; and
after said sufficient force has been applied, determining a capillary refill time from spectral changes in reflected light from the tissue detected by the optical sensor output.

15. A computer program comprising computer program code which is adapted, when said program is run on a computer, to implement the method of claim 14,
